# EUROPÄISCHE PATENTSCHRIFT

(11) **EP 0 818 468 B1**
(45) Veröffentlichungstag und Bekanntmachung des Hinweises auf die Patenterteilung: **28.08.2002**
(21) Anmeldenummer: 97109304.2
(22) Anmeldetag: 09.06.1997
(51) Int. Cl.: C08B 1/00, C08J 3/09, C12S 3/04

(54) **Verfahren zur Herstellung von Celluloselösungen in wasserhaltigem Aminoxid**
Process for preparing solutions of cellulose in aqueous amine oxide
Procédé pour la préparation de solutions de cellulose dans l'oxyde d'amine aqueux

(30) Priorität: 12.07.1996 DE 19628263
(43) Veröffentlichungstag der Anmeldung: 14.01.1998
(73) Patentinhaber: FRAUNHOFER-GESELLSCHAFT ZUR FÖRDERUNG DER ANGEWANDTEN FORSCHUNG E.V., 80636 München (DE)
(72) Erfinder: Schleicher, Harry, Prof.Dr., 14513 Teltow (DE); Weigel, Peter, Dr., 14532 Kleinmachnow (DE); Wetzel, Hendrik, Dr., 65510 Hünstetten (DE)

(56) Entgegenhaltungen:
- EP-A- 0 089 077
- EP-A- 0 473 545
- EP-B- 0 494 916
- DE-A- 4 439 149
- DE-A- 19 624 867

## Beschreibung

Die Erfindung betrifft ein Verfahren zur Herstellung von Celluloselösungen in wasserhaltigem Aminoxid.

Bei der Herstellung von Fasern, Folien und anderen Formen bzw. Gegenständen aus Cellulose nach dem Aminoxidverfahren ist es bisher bekannt, Zellstoff in bestimmten wasserhaltigen tertiären Aminoxiden unter Erhitzen zu lösen. Dabei wird meist so verfahren, daß Zellstoff in wäßriger Aminoxidlösung dispergiert und unter Erhitzen im Vakuum aus dieser Dispersion überschüssiges Wasser verdampft wird, wobei die Auflösung der Cellulose erfolgt. Diese Lösung wird dann durch Düsen gedrückt und nach Durchlaufen einer Luftstrecke in einem Fällmittel, das häufig eine wäßrige Aminoxidlösung ist, koaguliert.

Probleme treten hierbei dadurch auf, daß sowohl das Aminoxid als auch die darin gelöste Cellulose unter dem Einfluß der erhöhten Temperatur bei entsprechend erforderlicher Verweilzeit thermisch geschädigt werden können. Aus diesem Grund und aus dem Grund eines beschleunigten Durchsatzes soll die Cellulose sehr schnell und vollständig in Lösung mit dem wäßrigen Aminoxid gebracht werden.

Hierfür gibt es bereits verschiedene Vorschläge. So wird beispielsweise in der WO 95/11261 vorgeschlagen, eine Suspension aus Cellulose in wäßriger Aminoxidlösung einer Hochkonsistenzmahlung zu unterziehen und anschließend den Löseprozeß durchzuführen.

Eine andere Möglichkeit zur Verbesserung der Eigenschaften der Cellulose in bezug auf die Löslichkeit in wäßriger Aminoxidlösung kann man der EP 0 356 419 entnehmen. Danach soll der Zellstoff trocken gemahlen werden, was jedoch bei Abweichungen von den optimalen Mahlbedingungen dazu führt, daß durch die thermische Belastung eine Verhornung der Cellulose erfolgt, die die Löslichkeit der Cellulose negativ beeinflußt. Eine Alkalisierung mit anschließender Regenerierung oder eine hydrothermische Behandlung des Zellstoffs zur Verbesserung der Löslichkeit des Zellstoffs wird von E.Taeger, CH.Michels und A.Nechwatal in "Das Papier" 45(1991)784-788 bzw. in der DD 298 789 vorgeschlagen. Hierfür sind jedoch zusätzliche Verfahrensschritte erforderlich, die den Aufwand erheblich erhöhen.

Es ist auch bekannt, daß Enzyme nur in einem bestimmten Temperatur- und p_{H}-Bereich wirksam sind und bei Anwesenheit bestimmter Fremdsubstanzen besonders im Bereich erhöhter Konzentration inaktiv werden [Brockhaus ABC Chemie in zwei Bänden, VEB F.A. Brockhaus Verlag, Leipzig 1965, Bd.1, Seite 400; Herder-Lexikon der Biochemie und Molekularbiologie, Spektrum Akad. Verlag, Heidelberg, Berlin Oxford 1995, Bd.1, Seite 424-425].

Aus der DE 44 39 149 A1 ist die enzymatische Vorbehandlung von Cellulose und ihre anschließende Auflösung in einer Mischung aus N-Methylmorpholin-N-oxid und Wasser bekannt.

Ausgehend hiervon, ist es die Aufgabe der vorliegenden Erfindung, ein verbessertes Verfahren zur Herstellung von Celluloselösungen in wasserhaltigem Aminoxid vorzuschlagen, wobei eine beschleunigte Auflösung der Cellulose ohne wesentliche zusätzliche Maßnahmen erreicht werden soll.

Die Aufgabe wird durch die kennzeichnenden Merkmale des Anspruchs 1 gelöst. Vorteilhafte Weiterbildungen sind durch die Merkmale der Unteransprüche gekennzeichnet.

Erfindungsgemäß wird somit vorgeschlagen, Zellstoff in wäßrigen Aminoxidlösungen zu dispergieren und die so erhaltene Dispersion mit Xylanasen zu behandeln. Überraschenderweise wurde dabei festgestellt, daß sich in Dispersionen von Zellstoff in wäßrigen Lösungen von N-Methylmorpholin-N-oxid im Vergleich zu Dispersionen von Zellstoff in Wasser die Aktivität des Enzyms zwar verändert, daß aber trotzdem durch die Enzymbehandlung des Zellstoffs in der wäßrigen Aminoxidlösung die Eigenschaften des Zellstoffs so verändert werden, daß eine schnellere Auflösung des Zellstoffs in wasserhaltigem Aminoxid erreicht wird. Es können auch Xylanase enthaltende Enzymgemische verwendet werden. Besonders bevorzugt ist der Einsatz von Xylanase von Trichoderma viride.

Die Lösungsherstellung wird erfindungsgemäß in der Weise modifiziert, daß vor, während oder nach der Dispergierung des Zellstoffs in der wäßrigen Aminoxidlösung Xylanase oder Xylanase enthaltende Enzymgemische zugegeben werden. Nach einer zum Wirksamwerden des Enzyms erforderlichen Verweilzeit wird unter Erhitzen im Vakuum das überschüssige Wasser entfernt, wobei das Enzym zerstört wird und die Cellulose in Lösung geht. Das Enzym kann der wäßrigen Aminoxidlösung auch bereits vor der Zugabe des Zellstoffs zugefügt werden.

Die mögliche Behandlungstemperatur bei der Enzymeinwirkung ist vom Enzymtyp abhängig und sollte bei normalen Xylanasen etwa zwischen Raumtemperatur und 60°C liegen. Die notwendige Behandlungszeit hängt von der gewählten Enzymkonzentration und der Behandlungstemperatur sowie von der Konzentration der Aminoxidlösung ab. Es hat sich weiterhin als vorteilhaft herausgestellt, wenn die Zellstoffkonzentration in der enzymhaltigen Aminoxidlösung mehr als 3 % beträgt, bevorzugt 5 % bis 15 %. Die Enzymaktivität in der Dispersion sollte dabei oberhalb 0,1 U/g liegen. Unter Einhaltung dieser Verfahrensparameter werden besonders günstige Ergebnisse erzielt.

Nachfolgend soll das erfindungsgemäße Vorgehen an Beispielen näher erläutert werden:

### Beispiel 1

15,75 g Vorhydrolyse-Sulfat-Zellstoff (entsprechend 15 g atro Zellstoff wurden in 195,68 g 60%iger wäßriger Lösung von N-Methylmorpholin-N-oxid dispergiert und mit einer solchen Menge Xylanase (aus Trichoderma viride) versetzt, daß eine Aktivität von 4,35 U/g Aminoxidlösung erreicht wurde. Die Dispersion wurde nochmals homogenisiert und 23 Stunden bei 22°C aufbewahrt. Danach wurde unter Zusatz von 0,15 g Propylgallat zunächst bei 80°C in einem Kneter der NMMNO-Gehalt auf 80 % aufkonzentriert. 70 g dieses Slurrys wurden in einem Meßkneter 60 Minuten bei 70°C weiterbehandelt. Anschließend wurde die Temperatur auf 95°C erhöht, nach Erreichen dieser Temperatur durch Anlegen eines Vakuums innerhalb von 5 Minuten der Wassergehalt auf den NMMNO-Monohydratwert abgesenkt und dabei der Löseprozeß eingeleitet. Durch Verfolgen der Drehmomentänderung sowie durch Untersuchung der Proben in einem Polarisationsmikroskop erfolgte eine Bewertung des Auflösevorgangs. Der enzymbehandelte Zellstoff war nach 12 Minuten vollständig gelöst, während bei einem unter gleichen Bedingungen ohne Enzymzusatz durchgeführten Löseversuch die Auflösung erst nach 14 Minuten erfolgt war.

### Beispiel 2

Bei der Herstellung der Cellulose-Aminoxid-Wasser-Lösung entsprechend der Verfahrensweise des Beispiels 1 wurde die Dispersion des Zellstoffs in 60%iger wäßriger Aminoxidlösung mit einer solchen Menge Xylanase versetzt, daß eine Aktivität von 5,8 U/g Aminoxidlösung erreicht wurde. Das Gemisch wurde 26 Stunden bei 22°C aufbewahrt. In der Lösestufe war der Auflösevorgang nach 7,5 Minuten beendet.

### Beispiel 3

Bei der Herstellung der Cellulose-Aminoxid-Wasser-Lösung entsprechend der Verfahrensweise des Beispiels 1 wurde die Dispersion des Zellstoffs in 60%iger wäßriger Aminoxidlösung mit einer solchen Menge Xylanase versetzt, daß eine Aktivität von 7,25 U/g Aminoxidlösung erreicht wurde. Das Gemisch wurde 26 Stunden bei 22°C aufbewahrt. In der Lösestufe war der Auflösevorgang nach 10 Minuten beendet.

### Beispiel 4

Bei der Herstellung der Cellulose-Aminoxid-Wasser-Lösung entsprechend der Verfahrensweise des Beispiels 1 wurde die Dispersion des Zellstoffs in 60%iger wäßriger Aminoxidlösung mit einer solchen Menge Xylanase versetzt, daß eine Aktivität von 5,8 U/g Aminoxidlösung erreicht wurde. Das Gemisch wurde 2 Stunden bei 40°C aufbewahrt. In der Lösestufe war der Auflösevorgang nach 8,5 Minuten beendet.

### Beispiel 5

Bei der Herstellung der Cellulose-Aminoxid-Wasser-Lösung entsprechend der Verfahrensweise des Beispiels 1 wurde die Dispersion des Zellstoffs in 60%iger wäßriger Aminoxidlösung mit einer solchen Menge Xylanase (aus Trichoderma viride) und Cellulase (aus Aspergillus niger) versetzt, daß 5 U Xylanaseaktivität + 2 U Cellulaseaktivität/g Aminoxidlösung erreicht wurden. Das Gemisch wurde 24 Stunden bei 22°C aufbewahrt. In der Lösestufe war der Auflösevorgang nach 10,5 Minuten beendet.

## Patentansprüche

1. Verfahren zur Herstellung von Celluloselösungen
**dadurch gekennzeichnet, daß**
Zellstoff in einer wäßrigen Aminoxidlösung dispergiert und die so erhaltene Dispersion mit Xylanase oder Xylanase enthaltenden Enzymgemischen behandelt wird.

2. Verfahren nach Anspruch 1,
**dadurch gekennzeichnet, daß** die Enzymbehandlung bei Temperaturen zwischen 20°C und 60°C durchgeführt wird.

3. Verfahren nach Anspruch 1 oder 2,
**dadurch gekennzeichnet, daß** die Zellstoffkonzentration in der enzymhaltigen Aminoxidlösung zwischen 5 % und 15 % liegt.

4. Verfahren nach mindestens einem der Ansprüche 1 bis 3,
**dadurch gekennzeichnet, daß** die Enzymaktivität in der Dispersion so eingestellt wird, daß sie oberhalb 0,1 U/g liegt.

5. Verfahren nach Anspruch 4,
**dadurch gekennzeichnet, daß** Xylanase von Trichoderma viride verwendet wird.

## Claims

1. Method for producing cellulose solutions
***characterised in that***
cellulose pulp is dispersed in an aqueous amine oxide solution and the dispersion obtained thus is treated with xylanase or with enzyme mixtures which contain xylanase.

2. Method according to claim 1,
**characterised in that** the enzyme treatment is implemented at temperatures between 20 °C and 60 °C.

3. Method according to claim 1 or 2,
**characterised in that** the cellulose pulp concentration in the enzyme-containing amine oxide solution is between 5% and 15%.

4. Method according to at least one of the claims 1 to 3,
**characterised in that** the enzyme activity in the dispersion is set such that it is above 0.1 U/g.

5. Method according to claim 4,
**characterised in that** xylanase from Trichoderma viride is used.

## Revendications

1. Procédé de préparation de solutions de cellulose,
**caractérisé en ce qu'**
on disperse la pâte de cellulose dans une solution aqueuse d'aminoxyde et on traite la dispersion ainsi obtenue avec de la xylanase ou avec des mélanges d'enzymes contenant de la xylanase.

2. Procédé selon la revendication 1.
**caractérisé en ce qu'**
on effectue le traitement enzymatique à des températures comprises entre 20 et 60°C.

3. Procédé selon l'une quelconque des revendication 1 ou 2.
**caractérisé en ce que**
la concentration en pâte de cellulose dans la solution d'aminoxyde contenant un enzyme se situe entre 5 et 15 %.

4. Procédé selon au moins une des revendications 1 à 3,
caraclérisé en ce qu'
on règle l'activité enzymatique dans la dispersion de telle sorte qu'elle se situe au-dessus de 0,1 U/g.

5. Procédé selon la revendication 4,
**caractérisé en ce qu'**
on utilise une xylanase de trichoderma viride.
